(19) Europäisches Patentamt
European Patent Office
Office européen des brevets

(11) **EP 3 019 015 B1**

(12) **EUROPEAN PATENT SPECIFICATION**

(45) Date of publication and mention
of the grant of the patent:
**11.04.2018   Bulletin 2018/15**

(21) Application number: **14732883.5**

(22) Date of filing: **26.06.2014**

(51) Int Cl.:
*A01N 59/12* (2006.01)      *C11D 3/48* (2006.01)
*C11D 1/04* (2006.01)      *C11D 1/12* (2006.01)
*C11D 1/66* (2006.01)      *C11D 1/38* (2006.01)
*A61Q 17/00* (2006.01)      *A61K 8/20* (2006.01)
*C11D 1/14* (2006.01)      *C11D 1/22* (2006.01)

(86) International application number:
**PCT/EP2014/063469**

(87) International publication number:
**WO 2015/003911 (15.01.2015 Gazette 2015/02)**

(54) **DETERGENT COMPOSITION**

REINIGUNGSMITTELZUSAMMENSETZUNG

COMPOSITION DE DÉTERGENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.07.2013   EP 13176070**

(43) Date of publication of application:
**18.05.2016   Bulletin 2016/20**

(73) Proprietors:
• **Unilever N.V.
3013 AL Rotterdam (NL)**
Designated Contracting States:
**AL AT BE BG CH CZ DE DK EE ES FI FR GR HR
HU IS IT LI LT LU LV MC MK NL NO PL PT RO RS
SE SI SK SM TR**
• **Unilever PLC
London, Greater London EC4Y 0DY (GB)**
Designated Contracting States:
**CY GB IE MT**

(72) Inventors:
• **BISWAS, Sarmistha
Whitefield
Bangalore 560 066 (IN)**

• **NETHAJI, Alagirisamy
Whitefield
Bangalore 560 066 (IN)**
• **SAJI, Maya, Treesa
Whitefield
Bangalore 560 066 (IN)**
• **DASGUPTA, Anindya
Whitefield
Bangalore 560 066 (IN)**
• **SHRESTH, Rudra, Saurabh
Whitefield
Bangalore 560 066 (IN)**

(74) Representative: **Reijns, Tiemen Geert Pieter
Unilever Patent Group
Olivier van Noortstraat 120
3133 AT Vlaardingen (NL)**

(56) References cited:
**WO-A1-00/57703      WO-A1-01/11969
WO-A1-85/04184      WO-A1-97/07202
US-A- 3 338 837      US-A- 3 821 124**

**Description**

**Field of the invention**

The invention relates to cleaning compositions having anti-microbial benefits, in particular cleaning compositions having enhanced anti-microbial benefit because of the synergistic interaction between a sulphonated anionic surfactant and an iodine containing inorganic salt selected from potassium iodide and potassium iodate.

**Background of the invention**

Present day consumers appreciate detergent compositions for amongst others laundry cleaning and conditioning as well as household cleaning purposes; including hard surface cleaning and treatment composition, as well as dishwashing compositions. Several anti-microbial agents and compositions are known in the art, but many of them can be harmful to humans.

There is a long felt need for detergent compositions that are based on harmless and environmentally safe anti-microbial substances.

WO 2001/38626 discloses stain treatment compositions comprising surfactant and iodate at acidic pH. The iodate acts as an oxidising agent and gets reduced to iodine under acidic conditions. However acidic pH is not suitable for general detergent compositions, since stains and soil are better removed at alkaline pH. In WO2001/38626 it has been attempted to overcome the drawback of a low pH by adding bleach to the composition in a separate sub composition. This is undesirable for processing and packaging and needlessly increases the cost of the product. Additionally, bleach may cause damage to coloured fabrics and surfaces.

WO2008/137769 discloses warewashing compositions including a hardness ion (e. g. , magnesium and calcium ions) as a corrosion inhibitor. Such compositions can be used to reduce corrosion or etching of glass, porcelain and ceramic, it also relates to methods employing these warewashing compositions. WO2008/137769 particularly discloses a warewashing composition including a cleaning agent having a detersive amount of a surfactant, an alkaline source in an amount effective to provide a use composition having a pH of at least about 8 and a corrosion inhibitor in an amount sufficient for reducing corrosion of glass. The corrosion inhibitor as mentions includes a salt of calcium and/or magnesium; and iodide salts thereof are disclosed. However no mention is made to anti-microbial effect.

Similarly, US 2,599,140 discloses an iodine detergent for cleansing hands comprising a solvent mixture of a polyalkylene glycol and glycerine having in solution therein elemental iodine, an alkali metal iodide and a detergent compatible with the other ingredients, which detergent is an anionic sulphated synthetic detergent. However, this composition is slightly acidic and remains so on dilution and this is desirable as the iodine is more stable in slightly acidic composition and eliminates the risk of inactivation of the iodine with reaction with an alkali. Therefore, this composition would be undesirable in laundry and there still remains a need to provide an anti-microbial composition having neutral to alkaline pH that can be used with a variety of surfactants, including sulphonated anionic surfactants.

US 3,821,124 discloses detergent and shampoo compositions comprising an anionic, non-soap, non-sulphonate surfactant and iodate. However, sulphonated surfactants are especially preferred in many detergent compositions due to their low cost and high activity. It therefore remains to be desired to provide an anti-microbial composition having neutral to alkaline pH, which can be used with a variety of surfactants, including sulphonated and soap based anionic surfactants.

Similarly, US 3,338,837 relates to a highly germicidally-active iodinated detergent composition of high concentration comprising 4% to 15% of an organic detergent which may be anionic, cationic or non-ionic detergent, 10% to 30% of an organic halogen oxidant, 0.5% to 5% of an inorganic iodide and an inorganic alkalizer sufficient to raise the pH of the composition to between 7.8 to 8.4. This composition comprises a low amount of surfactant which does not give a high antimicrobial activity at alkaline conditions. It therefore remains to be desired to provide anti-microbial compositions having high antimicrobial activity at alkaline conditions and based on harmless and environmentally safe anti-microbial compositions.

Detergent compositions having a neutral to alkaline pH and that are based on harmless and environmentally safe anti-microbials providing good cleaning and improved anti-microbial benefits remain to be desired.

It is therefore an object of the present invention to provide a detergent composition with good cleaning and enhanced anti-microbial benefit at neutral to alkaline pH.

It is another object of the present invention to provide an anti-microbial composition based on harmless and environmentally safe antimicrobials.

Surprisingly, it has been found that at a neutral to alkaline pH, a synergistic anti-microbial activity may be obtained between a sulphonated anionic surfactant and an iodine containing inorganic salt selected from potassium iodate and potassium iodide.

**Summary of the invention**

Accordingly, in a first aspect, the present invention provides a detergent composition having anti-microbial benefits comprising 16% to 90%w of a sulphonated anionic surfactant wherein alkyl and acyl groups contain from 8 to 22 carbon atoms and 0.5% to 5%w of an iodine containing inorganic salt selected from potassium iodate and potassium iodide, wherein the pH of the composition is between 7 and 12.

In a second aspect, the invention provides a cleaning liquor comprising the composition according to the invention, wherein the iodine containing inorganic salt is in a concentration of between 0.1ppm to 150ppm.

In a third aspect, the invention provides an antimicrobial cleaning liquor, wherein the composition according to the invention is dosed in a concentration of between 1ppm and 5000ppm in the cleaning liquor.

In a fourth aspect, the invention provides a packaged cleaning composition, wherein the composition according to the invention is diluted with water in a ratio of between 1:10 to 1:1000 in a direct application container.

In a fifth aspect, the invention provides a method for rendering a substrate anti-microbial comprising the steps in sequence of preparing a 0.05% - 1% by weight solution of the composition according to the invention in water, allowing the substrate to be in contact with the solution for at least 1 min and; drying the substrate.

In a sixth aspect, the invention provides the use of a composition according to the invention for cleansing skin.

In a seventh aspect, the invention provides the use of 1ppm to 5000ppm of a composition according to the invention for treating a hard surface.

In the context of the present invention, the reference to "microbial" in the term anti-microbial includes bacteria, archaea, viruses, protozoa, fungi, algae or cysts.

In the context of the present invention, the reference to "substrate" typically means skin and household surfaces.

In the context of the invention, the most preferred applications are personal care and household cleaning while the present invention could be applicable in other fields like laundry as well.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

**Detailed description of the invention**

In a first aspect, the invention relates to a detergent composition comprising a sulphonated anionic surfactant wherein alkyl and acyl groups contain from 8 to 22 carbon atoms, and an iodine containing inorganic salt selected from potassium iodide and potassium iodate, wherein the pH of the composition is between 7 and 12.

Without wishing to be bound by a particular theory, the combination of specific surfactants with specific iodine salts provides an anti microbial effect.

Surfactant

The surfactant in the composition is selected from sulphonated anionic, surfactants.

In general, these surfactants are described in well known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981.

Anionic surfactants are the most preferred in laundry detergent compositions. Anionic surfactants for the detergent compositions which are used in the present invention are sulphonated anionic surfactants. Suitable sulphonated anionic surfactants include (usually) water-soluble alkali metal salts of organic sulphonates having alkyl radicals typically containing from about 8 to about 22 carbon atoms (the term alkyl being used to include the alkyl portion of higher acyl radicals), alkaryl sulphonates, alkanoyl isethionates, alkyl sulphosuccinates, N-alkoyl sarcosinates, linear alkyl benzene sulfonate (LAS) and alpha-olefin sulphonates. The alkyl and acyl groups contain from 8 to 22 carbon atoms, preferably 8 to 18 carbon atoms, still more preferably 12 to 15 carbon atoms and may be unsaturated.

Examples of suitable anionics include linear alkyl benzene sulfonate (LAS), Methyl Ester Sulphonate (MES), ammonium lauryl sulphosuccinate, sodium cocoyl isethionate, sodium lauroyl isethionate, and sodium N-lauryl sarcosinate.

The surfactant is present in the composition in a concentration of between 16% and 90% by weight, preferably at least 18% or even at least 20% by weight, but typically not more than 60%, still more preferably not more than 40% or even not more than 30% by weight of the composition.

The surfactant is present in the cleaning liquor in a concentration of between 2ppm and 2000ppm, preferably at least 20ppm, more preferably at least 50ppm, still more preferably at least 100ppm, but typically not more than 1500ppm or even not more than 1000ppm.

Iodine containing inorganic salt

The iodine containing inorganic salt according to the present invention is selected from potassium iodide and potassium iodate.

The iodine containing inorganic salt is present in the composition in a concentration of of between 0.5% and 5% by weight, preferably between 1% and 4%, still more preferably at least 2%, but typically not more than 3% by weight of the composition.

The iodine containing inorganic salt is present in the cleaning liquor in a concentration of between 0.1 ppm and 150ppm, preferably between 0.5ppm and 50ppm, more preferably between 1ppm and 35ppm.

pH of the composition

Anti-microbial activity of iodine salts is generally seen in an acidic pH. Therefore, iodine salt containing compositions having an acidic pH are commonly used as anti-microbial compositions. This is because antimicrobial agents such as iodine are more effective in acidic pH.

In the present invention, surprisingly the combination of a surfactant and the iodine containing inorganic salts has been found to have a synergistic antimicrobial activity at neutral to alkaline pH making it suitable for cleaning as well.

Therefore, the pH of the composition of the present invention is between 7 and 12. The best results are obtained when the pH of the composition is less than 11.

Other ingredients

The composition according to the invention may further comprise conventional ingredients including builder, electrolyte and/or fillers. When one or more of these ingredients are present, together they are typically present in the composition in a concentration of between 5% and 80% by weight of the total composition.

The composition may further comprise hydrotropes, soil release polymers, perfume, fluorescers, shading dyes, sequestrants, and anti re-deposition agents.

Method of use

For personal care and personal wash application of the composition of the invention, the composition according to the invention is preferably applied neat. In a preferred embodiment, the present invention relates to the use of the composition according to the invention for skin cleansing.

For hard surface cleaning, the composition according to the invention is preferably dosed to a concentration of between 1ppm and 5000ppm, preferably between 5ppm and 2000ppm, more preferably between 10ppm and 1000ppm and still more preferably between 10ppm and 500ppm in the cleaning liquor. In a preferred embodiment, the present invention relates to the use of 1ppm to 5000ppm of the composition according to the invention for treating a hard surface.

Product format

The composition may be used neat or diluted.

For hard surface cleaning products the composition is typically applied in a diluted form, directly to the surface. Such a composition is preferably diluted with water in a ratio of between 1:10 to 1:1000. Ideally the composition is packaged in a direct application container, such as a trigger spray dispenser.

For dishwashing purposes the composition according to the invention is applied neat. The composition according to the invention for dishwashing use is preferably a liquid, a paste, a powder or a shaped composition. Both manual dishwashing and machine dishwashing are considered in the context of the present invention.

For personal care and personal washing, the composition may be formulated in an aqueous base (water being carrier) e.g. products in gel format or in purely oil/solvent base e.g. products in anhydrous stick form or propellant containing products. However, most preferred product format are in liquid, solid, lotion or semisolid form for hand wash, face wash, body wash, or shaving applications.

### A method for rendering a substrate anti-microbial

A method for rendering a substrate anti-microbial comprising the steps in sequence of preparing a 0.05% - 1% by weight solution of the composition according to the invention in water, allowing the substrate to be in contact with the solution for at least 1 minute and drying the substrate.

The contact time required is dependent on the target microorganism. An enhanced antibacterial effect is achieved with a contact time of at least 1 minute for bacteria but in the case of viruses, a contact time of at least 5 minutes is required depending on the type and structure of the virus.

The invention will now be illustrated by means of the following non-limiting examples.

## Examples

### Materials

Iodine containing inorganic salt:

Potassium Iodide, Potassium Iodate (ex Sigma Aldrich)

|  | Surfactants: |
|---|---|
| Suphonated Anionic: | Sodium salt of dodecyl benzene sulphonate (DOBS) commonly referred to as linear alkyl sulphonate (LAS) (ex Rhodia Speciality Chemicals, Sigma Aldrich) |
| Suphated Anionic: | Sodium lauryl Ether Sulphate(SLES)(ex Galaxy Surfactants) |
| Cationic: | Benzalkonium chloride (BAC), (ex Sigma Aldrich Cas No. 63449-41-2) |
| Non-Ionic: | Linear Lauryl Alcohol Ethoxylate($EO_5$)(ex Galaxy Surfactants) |

### Method

Bacteria:

The anti-microbial effect of the composition according to the invention was demonstrated in by a bacterial plate test.

### Test solutions

Different test solution of surfactant and the iodine salt in water were prepared in the indicated concentrations. The test solutions as prepared had a pH of 7. Some experiments were done with the composition at pH 7, while for others the pH was adjusted to 10.5. The pH was adjusted with sodium carbonate solution (0.5 g/L $Na_2CO_3$ in water) or with sodium hydroxide.

Protocol: BS EN 1040 (modified with specific contact time)
Test bacteria: S.aureus ATCC 6538
E.hirae ATCC 10541

The test bacteria was grown overnight at 37°C on TSA plate. The grown culture colonies were resuspended in 0.8% saline solution. The culture cell density was adjusted to get the final count of $1*10^8$ cfu/ml, based on a 620nm optical density calibration chart (0.8 OD at 620nm). 9ml of the test solution was taken in a sterile sample container and 1ml of the test culture was added. After the specified contact time, 1ml of the above mixture was immediately neutralized in 9 ml D/E broth as commonly used in the art. This was again serially diluted in D/E broth and plated on TSA (a.k.a. Tryptic Soy Agar; commonly used in the art) in duplicates. In case of the control, 1 ml of test culture was added to 9 ml of saline and was serially diluted and plated on TSA. After solidification, the plates were incubated at 37°C for 48 hrs and the residual colonies were counted. All the antimicrobial experiments were performed under aseptic condition under laminar air flow and all the agar media and D/E dilution tubes were autoclaved (15 psig, 121 deg C, 15-20 min) before use.

In the examples below the log values of the residual colony forming units (cfu's) is given and compared. In comparing log value 1 point more reduction mean a 10 fold higher kill. So for instance when under comparative conditions the residual log value is 4 (i.e. 10000 cfu) and the inventive composition it is 2 (i.e. 100). That means that the end culture that is treated with the inventive composition has only 1% of the residual bacteria as the comparative composition. So 2 points on the log scale makes a big difference when it comes to hygiene.

Virus:

A standard EST protocol was (EN 14476) was followed for the evaluation of activity of antiviral actives in suspension test. Antiviral activity of actives was tested in clean conditions.

Clean conditions: To create clean conditions, 0.3g of bovine albumin fraction V was dissolved in 100 ml of water and sterilised by passing the solution through a 0.2 $\mu$m filter.

Preparation of test mixture: To prepare the test mixture, 1 ml of interfering substance (Bovine Albumin) was pipetted into a suitable container, followed by addition of 1ml of test virus suspension. To this, 8ml of product test solution was added. At the end of chosen contact time period, 100$\mu$l of the test mixture was added to resin column.

Centrifugation of microspin column (735 xg, 2 min). The elutant was diluted upto $10^{-6}$ in ice cold virus growth medium the infectivity of each dilution was measured by $TCID_{50}$ using Spaerman-Karber formula.

Titration of Virus: Monolayers of cells were grown in 96-well tissue-culture plates (Costar) at 37°C in an atmosphere of 95% air and 5% carbon dioxide ($CO_2$). Tenfold dilutions of virus suspension were prepared in virus growth medium. One hundred microlitres of each dilution was added to five replicate wells in 96-well tissue culture plates. Cell controls were included on each plate. Plates were incubated at 37°C in an atmosphere of 95% air/ 5% $CO_2$. Cultures were observed daily for viral cytopathic effect for a period of seven days after which they were discarded. The virus titre was calculated by $TCID_{50}$ (Tissue culture infective dose) using the Spaerman-Karber formula.

The formula is:

$$\text{Negative logarithm of the 50\% end point} = \text{Negative logarithm of the highest virus concentration used} - [(\text{Sum of \% affected at each dilution }/100-0.5)* (\text{lg of dilutions})]$$

**Example 1:** Anti-microbial effect of Potassium Iodide and sulphonated Anionic surfactant (Sodium LAS) on *S.aureus* at pH 7

In this example the improved anti-microbial efficacy of iodide (KI, potassium iodide) and a sulphonated anionic surfactant (LAS, Sodium linear alkylbenzene sulphonate) is demonstrated at pH 7 and compared to the individual components as well as the untreated control sample. The contact time was 1 minute.

|  | Residual cfu/ml | Residual log (cfu/ml) | Log reduction |
| --- | --- | --- | --- |
| *S.aureus* control | 1.60E+07 | 7.2 |  |
| 15ppm KI | 1.20E+07 | 7.1 | 0.1 |
| 100ppm LAS | 1.40E+06 | 6.1 | 1.1 |
| 300ppm LAS | 9.00E+04 | 5.0 | 2.2 |
| 15ppm KI + 100ppm LAS | 1.70E+03 | 3.2 | 4.0 |
| 15ppm KI + 300ppm LAS | 1.10E+03 | 3.0 | 4.2 |

As shown in the table above, the individual anti-microbial activity of KI and LAS alone is limited, while the combined efficacy is at least 2-log higher (i.e. 100 fold more reduction). The combined efficacy does not substantially increase further with increase in the concentration of LAS at pH 7, but the kill is still a 100 fold more than without the KI and 10000 fold more than without LAS.

**Example 2:** Anti-microbial effect of Potassium Iodate and sulphonated Anionic surfactant (Sodium LAS) on *S.aureus* at pH 7

In this example the improved anti-microbial efficacy of iodate ($KIO_3$, potassium iodate) and a sulphonated anionic surfactant (LAS, Sodium linear alkylbenzene sulphonate) is demonstrated at pH 7 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 15ppm KIO$_3$ | 1.20E+07 | 7.1 | 0.1 |
| 100ppm LAS | 1.40E+06 | 6.1 | 1.1 |
| 300ppm LAS | 9.00E+04 | 5.0 | 2.2 |
| 15ppm KIO$_3$+ 100ppm LAS | 2.80E+03 | 3.4 | 3.8 |
| 15ppm KIO$_3$+ 300ppm LAS | 1.70E+03 | 3.2 | 4.0 |

As shown in the table above, the individual anti-microbial activity of KIO3 and LAS alone is limited, while the combined efficacy is about 2-log higher (i.e. 100 fold more reduction).

**Example 3:** Anti-microbial effect of Potassium Iodide and sulphonated Anionic surfactant (Sodium LAS) on *S.aureus* at pH 10.5

In this example the improved anti-microbial efficacy of iodide (KI, potassium iodide) and a sulphonated anionic surfactant (LAS, Sodium linear alkylbenzene sulphonate) is demonstrated at pH 10.5 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 15ppm KI | 1.20E+07 | 7.1 | 0.1 |
| 100ppm LAS | 2.00E+06 | 6.3 | 0.9 |
| 300ppm LAS | 1.20E+05 | 5.1 | 2.1 |
| 15ppm KI +100ppm LAS | 1.00E+05 | 5.0 | 2.2 |
| 15ppm KI +300ppm LAS | 1.00E+04 | 4.0 | 3.2 |

As shown in the table above, the individual anti-microbial activity of KI and LAS alone is limited, while the combined efficacy is at least 1-log higher (i.e. 10 fold more reduction). The combined efficacy increases with increase in the concentration of LAS at pH 10.5.

**Example 4:** Anti-microbial effect of Potassium Iodate and sulphonated Anionic surfactant (Sodium LAS) on *S.aureus* at pH 10.5

In this example the improved anti-microbial efficacy of iodate (KIO$_3$, potassium iodate) and a sulphonated anionic surfactant (LAS, Sodium linear alkylbenzene sulphonate) is demonstrated at pH 10.5 and compared to the individual components as well as the untreated control sample. The contact time was 1 minute.

| | Residual cfu/ml | Residual log (cfu/ml) | Log reduction |
|---|---|---|---|
| *S.aureus* control | 1.60E+07 | 7.2 | |
| 15ppm KIO3 | 1.20E+07 | 7.1 | 0.1 |
| 100ppm LAS | 2.00E+06 | 6.3 | 0.9 |
| 300ppm LAS | 1.20E+05 | 5.1 | 2.1 |
| 15ppm KIO$_3$+100ppm LAS | 1.80E+05 | 5.3 | 1.9 |
| 15ppm KIO$_3$+300ppm LAS | 1.50E+04 | 4.2 | 3.0 |

As shown in the table above, the individual anti-microbial activity of KIO3 and LAS alone is limited, while the combined efficacy is about 1-log higher (i.e. 10 fold more reduction). The combined efficacy increases further with increase in the concentration of LAS at pH 10.5.

**Comparative Example 5:** Anti-microbial effect of Potassium Iodide and Cationic surfactant (BKC) on *S.aureus* at pH 7

In this example the improved anti-microbial efficacy of iodide (KI, potassium iodide) and a cationic surfactant (BKC, Benzalkonium chloride) is demonstrated at pH 7 and compared to the individual components as well as the untreated control sample. The contact time was 1 minute.

|  | Residual cfu/ml | Residual log (cfu/ml) | Log reduction |
|---|---|---|---|
| *S.aureus* control | 1.60E+07 | 7.2 |  |
| 40ppm BKC | 9.90E+05 | 6.0 | 1.2 |
| 15 ppm KI | 1.30E+07 | 7.1 | 0.1 |
| 15 ppm KI + 40ppm BKC | 1.10E+05 | 5.0 | 2.2 |
| 100 ppm BKC | 0.00E+00 |  | 7.2 |
| 100 ppm BKC + 15 ppm KI | 0.00E+00 |  | 7.2 |

As shown in the table above, the individual anti-microbial activity of KI and BKC alone is limited, while the combined efficacy is about 1-log higher (i.e. 10 fold more reduction).

**Comparative Example 6:** Anti-microbial effect of Potassium Iodate and Cationic surfactant (BKC) on *S.aureus* at pH 7

In this example the improved anti-microbial efficacy of iodate (KIO$_3$, potassium iodate) and a cationic surfactant (BKC, Benzalkonium chloride) is demonstrated at pH 7 and compared to the individual components as well as the untreated control sample. The contact time was 1 minute.

|  | Residual cfu/ml | Residual log (cfu/ml) | Log reduction |
|---|---|---|---|
| *S.aureus* control | 1.60E+07 | 7.2 |  |
| 40ppm BKC | 9.90E+05 | 6.0 | 1.2 |
| 15 ppm KIO3 | 1.30E+07 | 7.1 | 0.1 |
| 15ppm KIO3 + 40ppm BKC | 1.00E+05 | 5.0 | 2.2 |
| 100 ppm BKC | 0.00E+00 |  | 7.2 |
| 100ppm BKC+15ppm KIO3 | 0.00E+00 |  | 7.2 |

As shown in the table above, the individual anti-microbial activity of KIO3 and BKC alone is limited, while the combined efficacy is about 1-log higher (i.e. 10 fold more reduction).

**Comparative Example 7:** Anti-microbial effect of Potassium Iodide and sulphated Anionic surfactant (SLES) on *E.hirae* at pH 7

In this example the improved anti-microbial efficacy of iodide (KI, potassium iodide) and a sulphated anionic surfactant (SLES, Sodium lauryl ether sulfate) is demonstrated on a different bacteria, *E.hirae* at pH 7 and compared to the individual components as well as the untreated control sample. The contact time was 1 minute.

|  | Residual cfu/ml | Residual log (cfu/ml) | Log reduction |
|---|---|---|---|
| *E.hirae* control | 9.00E+07 | 8.0 |  |
| 100 ppm SLES | 6.50E+07 | 7.8 | 0.2 |
| 15 ppm KI | 8.50E+07 | 7.9 | 0.1 |

(continued)

| | Residual cfu/ml | Residual log (cfu/ml) | Log reduction |
|---|---|---|---|
| 100ppm SLES + 15ppm KI | 1.50E+07 | 7.2 | 0.8 |

As shown in the table above, the combined efficacy of KI and SLES is only about 0.5 log higher which is not preferred.

**Comparative Example 8:** Anti-microbial effect of Potassium Iodate and sulphated anionic surfactant (SLES) on *E.hirae* at pH 7

In this example the improved anti-microbial efficacy of iodate (KIO$_3$, potassium iodate) and an anionic surfactant (SLES, Sodium lauryl ether sulfate) is demonstrated on a different bacteria, *E.hirae* at pH 7 and compared to the individual components as well as the untreated control sample. The contact time was 1 minute.

| | Residual cfu/ml | Residual log (cfu/ml) | Log reduction |
|---|---|---|---|
| *E.hirae* control | 9.00E+07 | 8.0 | |
| 100 ppm SLES | 6.50E+07 | 7.8 | 0.2 |
| 15 ppm KIO3 | 9.00E+07 | 8.0 | 0.0 |
| 100ppm SLES +15ppm KIO3 | 1.50E+07 | 7.2 | 0.8 |

As shown in the table above, the combined efficacy of KIO3 and SLES is only about 0.5 log higher which is not preferred.

**Comparative Example 9:** Anti-microbial effect of Potassium Iodide and Non-ionic surfactants (EO5) on *S.aureus* at pH 7

In this example, the improved anti-microbial efficacy of iodide (KI, potassium iodide) and non-ionic surfactants (EO5) is demonstrated at pH 7 and compared to the individual components as well as the untreated control sample. The contact time was 1 minute.

| | Residual cfu/ml | Residual log (cfu/ml) | Log reduction |
|---|---|---|---|
| *S.aureus* control | 1.70E+07 | 7.2 | |
| 20 ppm KI | 1.60E+07 | 7.2 | 0.0 |
| 100ppm EO5 | 1.70E+07 | 7.2 | 0.0 |
| 100ppm EO5 + 20ppm KI | 8.50E+06 | 6.9 | 0.3 |

As shown in the table above, the individual anti-microbial activity of KI and the non-ionic surfactants (EO5) alone is limited, while the combined efficacy is at least 0.3-log higher. Even though non-ionic surfactants are less preferred in the present invention, there still exists a synergistic effect in the combination of KI and the non-ionic surfactants.

**Example 10:** Effect of increased concentration of surfactant on antimicrobial activity at pH 7.3

In this example, the concentration of the surfactant is increased substantially to check if there is an improved anti-microbial efficacy at high levels of the surfactant alone. The contact time was 1 minute.

| | Residual cfu/ml | Residual log (cfu/ml) | Log reduction |
|---|---|---|---|
| *S.aureus* control | 1.60E+07 | 7.2 | |
| 15ppm KI | 1.50E+07 | 7.2 | 0.0 |
| 150ppm KI | 1.50E+07 | 7.2 | 0.0 |
| 750 ppm NaLAS | 1.00E+05 | 5.0 | 2.2 |

(continued)

|  | Residual cfu/ml | Residual log (cfu/ml) | Log reduction |
|---|---|---|---|
| 1500ppm NaLAS | 8.60E+04 | 4.9 | 2.3 |
| 7500ppm NaLAS | 7.80E+04 | 4.9 | 2.3 |
| 15ppm KI + 750ppm NaLAS | 4.20E+02 | 2.6 | 4.6 |
| 15ppm KI + 1500ppm NaLAS | 2.00E+02 | 2.3 | 4.9 |
| 150ppm KI +7500ppm NaLAS | 0.00E+00 |  | 7.2 |

As shown in the table above, the anti-microbial activity of surfactant alone is limited even at high levels of the surfactant. This signifies that the antimicrobial effect of the composition of the present invention cannot be achieved with the individual ingredients alone even at high levels.

**Example 11:** Anti-microbial effect of Potassium Iodide and sulphonated Anionic surfactant (Sodium LAS) on *Polio virus* at pH 10.5

In this example, the improved anti-microbial efficacy of iodide (KI, potassium iodide) and a sulphonated anionic surfactant (LAS, Sodium linear alkylbenzene sulphonate) is demonstrated on a virus (polio virus) at pH 10.5 and compared to the individual components as well as the untreated control sample. The contact time was 15 minutes.

|  | N=1 | N=2 | N=3 | Residual log (avg) | Stdev | Log reduction |
|---|---|---|---|---|---|---|
| *Polio virus* control | 5.1 | 4.5 | 4.5 | 4.70 | 0.35 | - |
| 100ppm LAS | 4.1 | 2.9 | 3.3 | 3.43 | 0.61 | 1.27 |
| 20ppm KI | 4.2 | 3.7 | 3.7 | 3.87 | 0.29 | 0.83 |
| 100ppm LAS + 20ppm KI | 2.6 | 2.5 | 1.9 | 2.33 | 0.38 | 2.37 |

As shown in the table above, the individual anti-microbial activity of KI and LAS alone is limited, while the combined efficacy is at least 1-log higher (i.e. 10 fold more reduction).

**Comparative Example 12:** Anti-microbial effect of Potassium Iodide and Soap (Laurate) on *S.aureus* at pH 7

In this example the anti-microbial efficacy of iodide (KI, potassium iodide) and an anionic surfactant (soap) is demonstrated on *S.aureus* at pH 7 and compared to the individual components as well as the untreated control sample. The contact time was 1 minute.

|  | Residual cfu/ml | Residual log (cfu/ml) | Log reduction |
|---|---|---|---|
| *S.aureus* control | 1.60E+07 | 7.2 |  |
| 20 ppm KI | 1.30E+07 | 7.1 | 0.1 |
| 300ppm Laurate | 1.60E+07 | 7.2 | 0.0 |
| 20 ppm KI + 300ppm Laurate | 5.50E+06 | 6.7 | 0.5 |

As shown in the table above, the combined efficacy of KI and Laurate is negligible

**Claims**

1.  A detergent composition having anti-microbial benefits comprising:

a 16% to 90%w of a sulphonated anionic surfactant wherein alkyl and acyl groups contain from 8 to 22 carbon atoms; and

b 0.5% to 5%w of an iodine containing inorganic salt selected from potassium iodide and potassium iodate;

wherein the pH of the composition is between 7 and 12.

2. A detergent composition according to claim 1, wherein the sulphonated anionic surfactant is linear alkyl benzene sulfonate (LAS).

3. A composition according to claim 1 or 2, wherein the composition comprises 1% to 4%w of the iodine containing inorganic salt.

4. A composition according to anyone of the preceding claims, wherein the composition further comprises 5% to 80%w of one or more of conventional ingredients selected from builder, electrolyte and fillers.

5. A cleaning liquor comprising the composition according to anyone of claims 1 to 4, wherein the iodine containing inorganic salt is in a concentration of between 0.1 ppm and 150ppm.

6. Antimicrobial cleaning liquor, wherein the composition according to anyone of claims 1 to 4 is dosed in a concentration of between 1ppm and 5000ppm in the cleaning liquor.

7. A packaged cleaning composition, wherein the composition according to anyone of claims 1 to 4 is diluted with water in a ratio of between 1:10 to 1:1000 in a direct application container.

8. A method of rendering a substrate anti-microbial comprising the steps in sequence of:

a Preparing a 0.05% - 1% by weight solution of the composition according to anyone of claims 1 to 4 in water,
b Allowing the substrate to be in contact with the solution for at least 1 min and;
c drying the substrate.

9. Use of a composition according to anyone of claims 1 to 4 for cleansing skin.

10. Use of 1ppm to 5000ppm of a composition according anyone of claims 1 to 4, for treating a hard surface.


**Patentansprüche**

1. Reinigungszusammensetzung mit antimikrobiellen Vorteilen, umfassend:

a 16 bis 90 Gew.-% eines sulfonierten anionischen Tensids, wobei Alkyl- und Acyl-Gruppen von 8 bis 22 Kohlenstoffatome enthalten, und
b 0,5 bis 5 Gew.-% eines Iod enthaltenden anorganischen Salzes, ausgewählt aus Kaliumiodid und Kaliumiodat,

wobei der pH der Zusammensetzung zwischen 7 und 12 liegt.

2. Reinigungszusammensetzung nach Anspruch 1, wobei das sulfonierte anionische Tensid lineares Alkylbenzolsulfonat (LAS) ist.

3. Zusammensetzung nach Anspruch 1 oder 2, wobei die Zusammensetzung 1 bis 4 Gew.-% Iod enthaltendes anorganisches Salz umfasst.

4. Zusammensetzung nach irgendeinem der vorhergehenden Ansprüche, wobei die Zusammensetzung ferner 5 bis 80 Gew.-% eines oder mehrerer herkömmlicher Bestandteile umfasst, ausgewählt aus Builder, Elektrolyt und Füllstoffen.

5. Reinigungsflotte, umfassend die Zusammensetzung nach irgendeinem der Ansprüche 1 bis 4, wobei das Iod enthaltende anorganische Salz in einer Konzentration zwischen 0,1 ppm und 150 ppm vorliegt.

6. Antimikrobielle Reinigungsflotte, wobei die Zusammensetzung nach irgendeinem der Ansprüche 1 bis 4 in einer Konzentration zwischen 1 ppm und 5000 ppm in die Reinigungsflotte dosiert wird.

7.  Verpackte Reinigungszusammensetzung, wobei die Zusammensetzung nach irgendeinem der Ansprüche 1 bis 4 in einem Verhältnis zwischen 1:10 bis 1:1000 in einem Behälter zur direkten Anwendung mit Wasser verdünnt wird.

8.  Verfahren, mit dem ein Substrat antimikrobiell ausgefertigt wird, umfassend die Schritte in der Reihenfolge:

    a Herstellen einer 0,05 bis 1 gew.-%-igen Lösung der Zusammensetzung nach irgendeinem der Ansprüche 1 bis 4 in Wasser,
    b Belassen des Substrats in Kontakt mit der Lösung für mindestens 1 min und
    c Trocknen des Substrats.

9.  Verwendung einer Zusammensetzung nach irgendeinem der Ansprüche 1 bis 4 zur Hautreinigung.

10. Verwendung von 1 ppm bis 5000 ppm einer Zusammensetzung nach irgendeinem der Ansprüche 1 bis 4 zur Behandlung einer harten Oberfläche.

**Revendications**

1.  Composition de détergent ayant des bénéfices antimicrobiens comprenant :

    a de 16 % à 90 % en masse d'un tensioactif anionique sulfoné dans lequel les groupes alkyle et acyle contiennent de 8 à 22 atomes de carbone ; et
    b de 0,5 % à 5 % en masse d'un sel inorganique contenant de l'iode choisi parmi l'iodure de potassium et l'iodate de potassium ;

    où le pH de la composition est de 7 à 12.

2.  Composition de détergent selon la revendication 1, où le tensioactif anionique sulfoné est un benzènesulfonate d'alkyle linéaire (LAS).

3.  Composition selon la revendication 1 ou 2, où la composition comprend de 1 % à 4 % en masse du sel inorganique contenant de l'iode.

4.  Composition selon l'une quelconque des revendications précédentes, où la composition comprend de plus de 5 % à 80 % en masse d'un ou plusieurs ingrédients classiques choisis parmi un additif, un électrolyte et des charges.

5.  Liqueur de nettoyage comprenant la composition selon l'une quelconque des revendications 1 à 4, où le sel inorganique contenant de l'iode est présent dans une concentration de 0,1 ppm à 150 ppm.

6.  Liqueur de nettoyage antimicrobienne, où la composition selon l'une quelconque des revendications 1 à 4 est dosée dans une concentration de 1 ppm à 5 000 ppm dans la liqueur de nettoyage.

7.  Composition nettoyante emballée, où la composition selon l'une quelconque des revendications 1 à 4 est diluée avec de l'eau dans un rapport de 1:10 à 1:1 000 dans un récipient à application directe.

8.  Procédé rendant un substrat antimicrobien comprenant les étapes en séquence de :

    a préparation d'une solution à 0,05 % - 1 % en masse de la composition selon l'une quelconque des revendications 1 à 4 dans de l'eau,
    b laisser le substrat être en contact avec la solution pendant au moins 1 minute et ;
    c séchage du substrat.

9.  Utilisation d'une composition selon l'une quelconque des revendications 1 à 4 pour nettoyer la peau.

10. Utilisation de 1 ppm à 5 000 ppm d'une composition selon l'une quelconque des revendications 1 à 4, pour traiter une surface dure.

**EP 3 019 015 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200138626 A
- WO 2008137769 A
- US 2599140 A
- US 3821124 A
- US 3338837 A

### Non-patent literature cited in the description

- **SCHWARTZ ; PERRY.** Surface Active Agents. Interscience, 1949, vol. 1
- **SCHWARTZ ; PERRY ; BERCH.** SURFACE ACTIVE AGENTS. Interscience, 1958, vol. 2
- McCutcheon's Emulsifiers and Detergents. Manufacturing Confectioners Company
- **H. STACHE.** Tenside-Taschenbuch. Carl Hauser Verlag, 1981
- *CHEMICAL ABSTRACTS,* 63449-41-2